# EUROPÄISCHE PATENTSCHRIFT

(11) **EP 3 451 977 B1**
(45) Veröffentlichungstag und Bekanntmachung des Hinweises auf die Patenterteilung: **19.11.2025**
(21) Anmeldenummer: 17723308.7
(22) Anmeldetag: 02.05.2017
(51) Int. Cl.: A61F 2/80, A61F 2/50, A61F 5/01

(54) **ORTHOPÄDIETECHNISCHE EINRICHTUNG**
ORTHOPAEDIC DEVICE
DISPOSITIF ORTHOPÉDIQUE

(30) Priorität: 02.05.2016 DE 102016108145
(43) Veröffentlichungstag der Anmeldung: 13.03.2019
(73) Patentinhaber: Ottobock SE & Co. KGaA, 37115 Duderstadt (DE)
(72) Erfinder: LIDOLT, Klaus, 37115 Duderstadt (DE); WEHSELY-SWICZINSKI, Adam, 1012 Wien (AT)
(74) Vertreter: Gramm, Lins & Partner Patent- und Rechtsanwälte PartGmbB
(86) Internationale Anmeldenummer: PCT/EP2017/060389
(87) Internationale Veröffentlichungsnummer: WO 2017/191120

(56) Entgegenhaltungen:
- EP-A1- 0 401 910
- EP-A1- 0 401 910
- WO-A1-00/23016
- WO-A1-00/23016
- DE-U1- 202015 003 437
- DE-U1- 202015 003 437
- US-A1- 2006 079 964
- US-A1- 2006 079 964
- US-A1- 2010 036 505
- US-A1- 2010 036 505
- US-A1- 2015 190 262
- US-A1- 2015 190 262

## Beschreibung

Die Erfindung betrifft eine orthopädietechnische Einrichtung mit einem Grundkörper und wenigstens einem Gurt, der eingerichtet ist, den Grundkörper an einem Körperteil zu befestigen, durch wenigstens ein Führungselement geführt ist und ein freies Ende mit einem Befestigungselement zum Befestigen an einem korrespondierenden Gegenelement aufweist, wobei sich eine freie Länge des Gurtes von dem freien Ende bis zu dem Führungselement erstreckt.

Derartige orthopädietechnische Einrichtungen sind in einer Vielzahl unterschiedlicher Ausführungsformen bekannt und können beispielsweise als Orthesen oder Prothesen ausgebildet sein. Orthopädietechnische Einrichtungen verfügen über einen an einem Körperteil anzulegende Grundkörper, der vorzugsweise ein Schalen- oder Schienenelement aufweist, das durch wenigstens einen Gurt am jeweiligen Körperteil festgelegt wird. Ein Grundkörper kann jedoch auch aus einem flexiblen und vorzugsweise elastischen Material, beispielsweise einem Textil hergestellt sein. Dabei ist es möglich, dass der Gurt selbst ausschließlich der Befestigung dient oder selbst weitere stützende oder schützende Aufgaben übernimmt. Der Gurt selbst kann elastisch oder unelastisch ausgebildet sein. Zudem ist es möglich, dass der Gurt das jeweilige Körperteil, an dem der Gegenstand anzuordnen ist, vollständig oder nur teilweise umfasst oder umgreift.

Der jeweilige Gurt ist in der Regel durch ein Führungselement geführt, das beispielsweise eine Schlinge, eine Schlaufe, ein Schlitz oder ein Umlenkelement sein kann. Selbstverständlich sind auch andere Arten von Führungselementen denkbar. Am freien Ende des Gurtes befindet sich wenigstens ein Befestigungselement, das beispielsweise eine Schnalle, ein Formschlusselement, beispielsweise ein Klettverschlusselement, ein Clipselement oder ein sonstiges Befestigungselement sein kann. Mit diesem Befestigungselement kann das freie Ende an einem jeweiligen korrespondierenden Gegenelement, das zum Befestigungselement passend ausgebildet ist, angeordnet werden. Unter dieser Ausgestaltung fällt beispielsweise auch ein Gurt, an dessen freiem Ende sich beide zueinander korrespondierend ausgebildete Klettverschlusselemente befinden, so dass der Gurt beispielsweise durch eine Öse oder Schlaufe, die in diesem Fall das Gegenelement bildet, hindurchgefädelt und anschließend auf sich selbst festgelegt wird. Selbstverständlich sind auch andere Möglichkeiten einer derartigen Anordnung denkbar. Andere Befestigungselemente können beispielsweise Schnallen, Clipselemente, Knöpfe oder Druckknöpfe sein, die an einem entsprechenden Gegenelemente befestigt werden können.

Im angelegten Zustand der orthopädietechnischen Einrichtung wird der Gurt folglich benötigt, um beispielsweise die Einrichtung selbst an einem Körperteil des Trägers anzuordnen oder aus orthopädietechnischer Sicht vorteilhafte und gewünschte Kräfte, beispielsweise Druck- oder Zugkräfte, auf ein jeweiliges Körperteil aufbringen zu können. Dazu muss der Gurt selbstverständlich eine ausreichende Länge aufweisen, die es ermöglicht, das Befestigungselement am freien Ende des Gurtes am jeweiligen Gegenelement anzuordnen. Insbesondere bei Gurten, die beispielsweise um den Rumpf eines Trägers herum gelegt werden sollen aber auch in anderen Funktionen des Gurtes, muss der Gurt folglich eine nicht unerhebliche Länge aufweisen, um die gewünschten Funktionen erfüllen zu können.

Befindet sich die orthopädietechnische Einrichtung nicht im angelegten Zustand, ist in der Regel das Befestigungselement nicht am jeweiligen Gegenelement angeordnet, sondern das freie Ende des Gurtes baumelt frei vom Rest der jeweiligen orthopädietechnischen Einrichtung herab. Dies birgt die Gefahr, dass sich gegebenenfalls mehrere vorhandene Gurte ineinander verheddern oder ein entsprechender Gurt an einem anderen Gegenstand hängen bleibt, sich um ihn herum wickelt oder auf andere Weise mit ihm zusammenwirkt. Handelt es sich bei dem Befestigungselement beispielsweise um einen Teil eines Magnetverschlusses, besteht die Gefahr, dass das Befestigungselement mit anderen metallischen Gegenständen, beispielsweise Möbeln oder Türrahmen, so zusammenwirkt, dass das freie Ende des Gurtes am jeweiligen Gegenstand hängenbleibt. Handelt es sich bei dem Befestigungselement um einen Teil eines Klettverschlusselementes besteht die gleiche Gefahr bei textilen Gegenständen, wie beispielsweise Kleidungsstücken oder Sitzbezügen. Hinzukommt, dass insbesondere bei einer großen freien Länge des Gurtes der Gurt auch beim Anlegen der orthopädietechnischen Einrichtung stören kann, da er unkontrolliert schwingt und baumelt und so das Anlegen der orthopädietechnischen Einrichtung an ein Körperteil stört, da er sich beispielsweise zwischen einem Schalen- oder Schienenelement und dem entsprechenden einzulegenden oder anzuordnenden Körperteil befindet, oder beim Anlegen des Gurtes das freie Ende aufgrund der unkontrollierten Bewegung des Gurtes nicht oder nur schwierig durch den Träger der orthopädietechnischen Einrichtung zu greifen ist.

Aus der DE 20 2015 003 437 U1 ist eine Einrichtung bekannt, bei der durch einen Gurt ein Druck eingestellt werden kann, der durch eine Pelotte aufgebracht wird. Die US 2015/0190262 A1 beschreibt eine Einrichtung, bei der ein spannbarer Gurt verwendet wird, der gespannt oder entspannt werden kann. Der Gurt wird jedoch an keinem Ende gelöst. Die US 2006/0079964 A1 beschreibt eine Einrichtung, bei der ein Liner, der über einen Amputationsstumpf gezogen wird, durch einen Gurt an einem Prothesenschaft befestigt wird.

Der Erfindung liegt daher die Aufgabe zugrunde, eine orthopädietechnische Einrichtung so weiterzuentwickeln, dass diese Nachteile aus dem Stand der Technik behoben oder zumindest abgemildert werden.

Die Erfindung löst die gestellte Aufgabe durch eine orthopädietechnische Einrichtung gemäß Anspruch 1.

Die freie Länge des Gurtes entspricht dabei in aller Regel dem Teil des Gurtes, der frei und unkontrolliert beweglich ist sofern das Befestigungselement nicht am korrespondierenden Gegenelement befestigt ist. Es ist der Teil des Gurtes, der in diesem Fall von der orthopädietechnischen Einrichtung frei herunterhängt und unkontrolliert baumelt. Mit der erfindungsgemäßen Ausgestaltung der orthopädietechnischen Einrichtung ist sichergestellt, dass der Gurt in die zweite Position gebracht werden kann, in der diese freie Länge reduziert wird. Damit wird die Bewegungsfreiheit des freien Endes stark eingeschränkt, wodurch die Probleme aus dem Stand der Technik zumindest abgemildert, und bei sehr starker Reduzierung der freien Länge nahezu vollständig gelöst werden können.

Verfügt der Gurt in der zweiten Position über eine sehr geringe freie Länge ist nahezu ausgeschlossen, dass sich der Gurt störend beim Anlegen der orthopädietechnischen Einrichtung zwischen einen Teil der Einrichtung und einem in diesen Teil einzulegenden oder an diesem Teil anzuordnenden Körperteil des Trägers der orthopädietechnischen Einrichtung bewegt. Zudem wird nahezu sicher verhindert, dass der Gurt mit anderen Gegenständen interagiert und zusammenwirkt und so beispielsweise an diesen Gegenständen anhaftet oder hängenbleibt. Zudem ist beim Anlegen der orthopädietechnischen Einrichtung für den Träger der Einrichtung klar, an welcher Stelle sich das freie Ende, an dem das Befestigungselement angeordnet ist, befindet. Ein Suchen oder umständliches Tasten nach dem freien Ende des Gurtes entfällt somit.

Durch die Überführung des Gurtes aus der ersten Position in die zweite Position wird folglich nicht nur die freie Länge des Gurtes reduziert. Vorzugsweise wird der Gurt beim Verschieben aus der ersten Position in die zweite Position durch das Führungselement hindurchbewegt, so dass die freie Länge des Gurtes, also der Teil des Gurtes zwischen seinem freien Ende und dem Führungselement, in seiner Länge reduziert wird. Der Teil des Gurtes, der dabei durch das Führungselement hindurch bewegt wird und somit nicht mehr zur freien Länge beiträgt, liegt erfindungsgemäß zumindest nahezu vollständig, vorteilhafterweise vollständig an einem anderen Bauteil der orthopädietechnischen Einrichtung an. Alternativ oder zusätzlich kann er in eine dafür vorgesehene Öffnung oder einem dafür vorgesehenen Hohlraum verschoben werden. Alternativ oder zusätzlich dazu kann auch ein Teil des Gurtes auf eine dafür vorgesehen Wickeleinrichtung, beispielsweise eine Rolle aufgewickelt werden. In einer bevorzugten Ausgestaltung wird durch jede einzelne oder eine Kombination mehrerer oder aller dieser Maßnahmen erreicht, dass durch das Überführen des Gurtes aus der ersten Position in die zweite Position keine neuen Schlaufen oder von der orthopädietechnischen Einrichtung herabbaumelnden Enden oder Bereiche des Gurtes erzeugt werden, und/oder bestehende Schlaufen nicht verlängert oder vergrößert werden. Dadurch wird die Gefahr, dass sich der Gurt verheddert oder an einem nicht zur orthopädietechnischen Einrichtung gehörenden Gegenstand hängen bleibt, stark reduziert.

Das wenigstens eine Führungselement kann an dem Gurt befestigt oder in ihm integriert sein. Dies ist insbesondere dann von Vorteil, wenn der Gurt auf sich selbst zurückgelegt und somit durch das Führungselement hindurchgeführt werden kann. Eine Befestigung der beiden übereinanderliegenden Bestandteile des Gurtes findet dabei jedoch nicht statt, um weiterhin eine Bewegung des Gurtes durch das Führungselement sicherzustellen.

Vorzugsweise weist der Grundkörper wenigstens eine Schale und/oder eine Schiene auf. Vorzugsweise ist das wenigstens eine Führungselement an der Schale und/oder Schiene angeordnet oder darin integriert.

**In** einer bevorzugten Ausgestaltung ist der Gurt von der ersten Position in die zweite Position bringbar, in dem eine Zugkraft auf ein zweites Ende des Gurtes aufgebracht wird, das dem freien Ende gegenüberliegt. Der Gurt wird auf diese Weise durch das Führungselement hindurchgezogen, so dass das freie Ende näher am Führungselement positioniert wird. Gleichzeitig wird dadurch die freie Länge des Gurtes reduziert. Beim Anlegen der orthopädietechnischen Einrichtung befindet sich der Gurt vorteilhafterweise in der zweiten Position, um möglichst wenig beim Anlegen zu stören. Soll der Gurt dann in die erste Position überführt werden, um das Befestigungselement am entsprechenden Gegenelement befestigen zu können, kann einfach eine Zugkraft auf das freie Ende des Gurtes ausgeübt werden. Dies geschieht beispielsweise indem der Träger der orthopädietechnischen Einrichtung beim Anlegen das freie Ende des Gurtes greift, daran zieht und anschließend das Befestigungselement am Gegenelement befestigt.

Es hat sich als vorteilhaft herausgestellt, wenn die Einrichtung eine Zugeinrichtung aufweist, durch die die Zugkraft auf das zweite Ende aufbringbar ist. Diese Zugeinrichtung kann vorteilhafterweise eine Zugfeder sein. Alternativ oder zusätzlich dazu weist die Zugeinrichtung eine Wickeleinrichtung auf, durch die der Gurt aufwickelbar ist, wobei vorzugsweise das Führungselement Teil der Wickeleinrichtung ist.

Alle vorteilhafterweise verwendbaren Zugeinrichtungen sind jedoch in der Lage, automatisch, also ohne separate Betätigung, eine Zugkraft auf das zweite Ende aufzubringen. Sobald also beispielsweise zum Ablegen der orthopädietechnischen Einrichtung das Befestigungselement vom entsprechenden Gegenelement gelöst und anschließend beispielsweise losgelassen wird, sorgt die für die Zugeinrichtung auf das zweite Ende aufgebrachte Zugkraft dafür, dass der Gurt automatisch aus der ersten Position in die zweite Position überführt wird und die freie Länge auf diese Weise reduziert wird. Selbstverständlich ist es auch möglich, dies durch eine Verriegelungseinrichtung zu verhindern, die durch ein Betätigungselement, beispielsweise ein Druckknopf, betätigt und entriegelt werden kann.

Beim Ablegen der orthopädietechnischen Einrichtung kann folglich das Befestigungselement vom Gegenelement gelöst werden und die von der Zugeinrichtung auf das zweite Ende des Gurtes aufgebrachte Zugkraft entfaltet ihre Wirkung erst, nachdem die Verriegelungseinrichtung entriegelt wurde. Dann jedoch wird der Gurt durch die aufgebrachte Zugkraft aus der ersten Position in die zweite Position gebracht. Die Verriegelungseinrichtung kann so ausgebildet sein, dass sie zwar manuell entriegelt werden muss, aber automatisch verriegelt, sobald der Gurt aus der zweiten Position in die erste Position gebracht wird. Selbstverständlich ist auch das manuelle Verriegeln einer entsprechenden Verriegelungsvorrichtung möglich.

Wird als Zugeinrichtung zumindest auch eine Wickeleinrichtung verwendet, ist vorteilhafterweise das zweite Ende des Gurtes an einer rotierbar gelagerten Achse oder Welle der Wickeleinrichtung befestigt. Diese wird bei Betätigung der Zugeinrichtung in Rotation versetzt und wickelt auf diese Weise den Gurt vom zweiten Ende her auf, so dass die freie Länge reduziert wird. Diese Ausgestaltung hat den Vorteil, dass eine platzsparende und fehlerunanfällige Lagerung des in die zweite Position versetzten Gurtes ermöglicht wird. Die Gefahr, dass sich der Gurt in der zweiten Position verheddert oder verknotet wird auf diese Weise stark reduziert.

Insbesondere für den Fall, dass eine Wickeleinrichtung verwendet wird, die einen Teil des Gurtes aufwickeln kann, um den Gurt aus der ersten Position in die zweite Position zu bringen, ist es von Vorteil, das Führungselement als separates Bauteil und nicht als Teil der Wickeleinrichtung auszubilden. In einer bevorzugten Ausgestaltung kann das Führungselement folglich als das Element definiert werden, das in Richtung auf das freie Ende des Gurtes den Gurt als letztes zumindest teilweise, vorteilhafterweise jedoch vollständig überspannt. Wird der Gurt in die zweite Position gebracht, in dem er in eine dafür vorgesehene Aufnahmeeinrichtung oder ein Volumen verschoben wird, kann dies beispielsweise durch einen Öffnungsschlitz geschehen, der in diesem Fall ebenfalls als Führungselement angesehen werden kann und als solches wirkt.

Selbstverständlich kann ein weiteres zusätzliches Führungselement Teil der Wickeleinrichtung sein. Auf diese Weise kann sichergestellt werden, dass es beim Einziehen des Gurtes in die Wickeleinrichtung nicht zu einem Umschlagen eines Gurtes kommt, wodurch eine ordnungsgemäße Funktion der Wickeleinrichtung nicht mehr gewährleistet werden könnte. Wichtig ist jedoch, wenigstens ein Führungselement vorzusehen, das nicht Teil der Wickeleinrichtung ist.

Vorteilhafterweise ist der Gurt von der ersten Position in die zweite Position bringbar, indem er zumindest teilweise in eine Aufnahmeeinrichtung verschoben wird. Dadurch ist sichergestellt, dass der Gurt in der zweiten Position in geordneter Weise verstaut wird und sich nicht verheddern oder verknoten kann. Zudem wird verhindert, dass der durch das Führungselement hindurchgezogene Anteil des Gurtes oder ein anderer Anteil, der durch das Verschieben des Gurtes aus der ersten Position in die zweite Position bewegt an anderer Stelle störend beispielsweise auf den Träger der orthopädietechnischen Einrichtung oder das Anlegen der orthopädietechnischen Einrichtung einwirkt. Die Aufnahmeeinrichtung befindet sich vorteilhafterweise an einem starren Bauteil, beispielsweise einer Schiene oder einer Schale der orthopädietechnischen Einrichtung. Hier kann sie konstruktiv einfach und dennoch leicht zugänglich angeordnet werden. Vorteilhafterweise ist die Aufnahmeeinrichtung so ausgebildet, dass sie geöffnet werden kann. Auf diese Weise ist der Gurt auch dann zugänglich, wenn er sich in der zweiten Position befindet, was insbesondere dann von Vorteil ist, wenn Störungen auftreten, der Gurt untersucht oder durch einen anderen Gurt ersetzt werden soll.

Vorzugsweise verfügt die Aufnahmeeinrichtung über mehrere Stege, die den Gurt quer zu seiner Längsrichtung von dem freien Ende zu dem zweiten Ende überspannen, wenn sich der Gurt in der zweiten Position befindet. Die Längsrichtung des Gurtes erstreckt sich von dem freien Ende zu dem zweiten Ende. Die Stege überspannen den Gurt quer zu dieser Richtung. Dabei bedeutet quer nicht notwendigerweise einen 90° Winkel. Es ist ausreichend, wenn ein Ende der Stege auf der einen Seite, beispielsweise in Längsrichtung links zum Gurt, angeordnet ist, während das andere Ende der Stege auf der gegenüberliegenden Seite, im vorliegenden Beispiel also in Längsrichtung rechts vom Gurt, angeordnet wird. Durch die Verwendung mehrerer Stege wird einerseits gewährleistet, dass der Gurt in der Aufnahmeeinrichtung verbleibt und nicht zumindest teilweise störend aus der Aufnahmeeinrichtung herausbewegen kann, und andererseits erreicht, dass zumindest ein Großteil des in der Aufnahmeeinrichtung enthaltenen Anteils des Gurtes weiterhin sichtbar und somit beispielsweise auch Probleme oder Fehler sowie Beschädigungen zumindest optisch untersucht werden kann.

Ein Steg oder ein anderer Teil der Aufnahmeeinrichtung kann als Führungselement verwendet werden.

Wird der Gurt in eine Aufnahmeeinrichtung hineingezogen, liegt er nahezu vollflächig an dem Schienen- oder Schalenelement der orthopädietechnischen Einrichtung an. Die Aufnahmeeinrichtung erstreckt sich folglich vorzugsweise direkt an oder in dem Schalen- oder Schienenelement, so dass der zusätzliche Bauraum der orthopädietechnischen Einrichtung, der durch die Aufnahmeeinrichtung benötigt wird, nur gering ist. Dies ist anders, wenn eine Wickeleinrichtung verwendet wird, da diese sich nur in einem relativ begrenzten Bereich der orthopädietechnischen Einrichtung befindet, hier jedoch einen relativ großen zusätzlichen Bauraum erfordert. Es ist daher von Vorteil, diese Wickeleinrichtung an einer Stelle der orthopädietechnischen Einrichtung zu positionieren, wo der zusätzliche Bauraum zur Verfügung steht und die Wickeleinrichtung möglichst wenig stört. Daher ist es von Vorteil, in diesem Fall wenigstens ein Führungselement vorzusehen, das nicht Teil der Wickeleinrichtung ist.

In einer bevorzugten Ausgestaltung verfügt die Aufnahmeeinrichtung über wenigstens einen Längsschlitz entlang der Längsrichtung des Gurtes, durch den ein Griffelement des Gurtes hindurchragt. Dieses Griffelement, das beispielsweise als Vorsprung ausgebildet sein kann, steht somit nach außen aus der Aufnahmeeinrichtung hervor und ist somit für den Träger der orthopädietechnischen Einrichtung zugänglich. In einer konstruktiven sehr einfachen Ausgestaltung ist es daher nicht notwendig, eine zusätzliche Zugeinrichtung vorzusehen. Soll der Gurt von der ersten Position in die zweite Position gebracht werden, muss der Träger der orthopädietechnischen Einrichtung lediglich das Griffelement des Gurtes ergreifen und entlang des Längsschlitzes verschieben. Auf diese Weise übt er selbst die Zugkraft auf den Gurt aus, die vorteilhafterweise auch am zweiten Ende des Gurtes aufgebracht wird. Dadurch wird der Gurt in die zweite Position verschoben.

Vorzugsweise verfügt der Gurt über ein Anschlagselement, das an einem Anschlag der orthopädietechnischen Einrichtung anliegt, wenn sich der Gurt in der ersten Position befindet. Wird der Gurt aus der zweiten Position in die erste Position gebracht, indem beispielsweise auf das freie Ende des Gurtes eine Zugkraft beispielsweise durch den Träger der orthopädietechnischen Einrichtung, aufgebracht wird, verschiebt sich der Gurt bis zu dem Moment, an dem das Anschlagselement des Gurtes an dem Anschlag der Einrichtung anliegt. Für den Träger der orthopädietechnischen Einrichtung ergibt dies ein sicheres Zeichen dafür, dass sich der Gurt nun in der ersten Position befindet und daher die freie Länge ausreichend groß ist, das Befestigungselement im angelegten Zustand der orthopädietechnischen Einrichtung an dem entsprechend korrespondierend ausgebildeten Gegenelement angeordnet werden kann. Dies ist insbesondere dann von Vorteil, wenn der Gurt selbst elastisch ausgebildet ist oder zumindest einen elastischen Anteil aufweist. Verhakt sich beispielsweise der Gurt auf dem Weg von der zweiten Position in die erste Position, so dass für den Träger der orthopädietechnischen Einrichtung, der eine Zugkraft auf das freie Ende aufbringt, der Eindruck entsteht, der Gurt hätte bereits seine erste Position erreicht, könnte dies dazu führen, dass um die noch fehlende freie Länge auszugleichen der elastische Anteil des Gurtes überdehnt wird, um das Befestigungselement am korrespondierenden Gegenelement zu befestigen. Dies hätte einerseits eine übermäßig starke mechanische Belastung des elastischen Gurtelementes zur Folge und würde andererseits eine zu große Kraft und einen zu großen Druck auf ein Körperteil des Trägers der orthopädietechnischen Einrichtung aufbringen. Dies kann sicher vermieden werden, wenn der Träger der Einrichtung dafür sorgt und darauf achtet, dass das Anschlagselement am Anschlag anliegt.

Vorzugsweise ist die Position des Anschlags und/oder des Anschlagelementes einstellbar. Auf diese Weise kann die freie Länge, die der Gurt in der ersten Position aufweist, variiert werden. Insbesondere bei elastisch ausgebildeten Gurten aber auch bei einer unelastischen Ausgestaltung ist es dadurch möglich, die durch den Gurt auf das jeweilige Körperteil aufgebrachte Kraft zu dosieren und an die individuellen Gegebenheiten und Bedürfnisse des Trägers anzupassen.

Vorteilhafterweise verfügt der Gurt über ein Anschlagselement, das an einem Anschlag der orthopädietechnischen Einrichtung anliegt, wenn sich der Gurt in der zweiten Position befindet. Dadurch wird verhindert, dass der Gurt zu weit eingezogen wird, oder sich vollständig aufrollt, wenn der Gurt geöffnet und in die zweite Position gebracht wird. Es wird folglich sichergestellt, dass der Gurt vom Träger der Orthese noch gut gegriffen und insbesondere wieder in die erste Position gebracht wird. Das Anschlagselement kann vorteilhafterweise als Griffelement, beispielsweise als Schlaufe, ausgebildet sein, welches zum Herausziehen des Gurtes, also zum Überführen des Gurtes aus der zweiten Position in die erste Position, verwendet wird.

**In** einer bevorzugten Ausgestaltung befindet sich am freien Ende des Gurtes ein Greifelement, beispielsweise eine Schlaufe oder ein Griffstück. Auf diese Weise kann der Gurt leicht gegriffen, die Befestigung am Gegenelement leicht hergestellt oder geöffnet werden. Dies ist insbesondere für Personen von Vorteil, die beispielsweise aufgrund körperlicher Einschränkungen nicht oder nur bedingt in der Lage sind, die erforderliche Zugkraft aufzubringen.

Vorteilhafterweise ist das wenigstens ein Führungselement lösbar angeordnet und vorzugsweise in unterschiedlichen Positionen, vorteilhafterweise stufenlos, an dem Schalen- oder Schienenelement oder dem Gurt befestigbar. Damit ist eine individuelle Anpassung an die jeweiligen Gegebenheiten und gegebenenfalls sogar an dem Patienten, der die orthopädietechnische Einrichtung verwendet, möglich.

Zudem wird es möglich, das Führungselement möglichst weit beispielsweise von einer Aufnahmeeinrichtung oder einer Wickeleinrichtung entfernt anzuordnen. Dadurch wird die Führung des Gurtes deutlich verbessert und auch die Gleiteigenschaften des Gurtes vorteilhaft beeinflusst. Auch dies trägt dazu bei, eine Wickeleinrichtung dort an der orthopädietechnischen Einrichtung anzuordnen, wo sie den Träger möglichst wenig stört. So wird beispielsweise bei einer Knieorthese eine Wickeleinrichtung vorteilhafterweise lateral oder posterior angeordnet, da sie an diesen Stellen insbesondere beim Gehen oder Laufen mit der Orthese am wenigsten stört. Eine Anordnung medial hätte diesen Nachteil, wäre ohne zusätzliches Führungselement jedoch kaum zu vermeiden. Bei einer Schulterorthese beispielsweise kann durch das zusätzliche Führungselement, das nicht Teil der Wickeleinrichtung ist, vermieden werden, die Wickeleinrichtung im Achselbereich, beispielsweise unter der Achselhöhle, anzuordnen.

Mit Hilfe der beiliegenden Figuren werden nachfolgend einige Ausführungsbeispiele der vorliegenden Erfindung näher erläutert. Es zeigt:
- Figuren 1 bis 4 -: eine orthopädietechnische Einrichtung gemäß einem ersten Ausführungsbeispiel der vorliegenden Erfindung in unterschiedlichen Zuständen,
- Figuren 5 bis 8 -: eine Einrichtung gemäß einem weiteren Ausführungsbeispiel der vorliegenden Erfindung in unterschiedlichen Zuständen,
- Figuren 9 bis 12 -: eine Einrichtung gemäß einem dritten Ausführungsbeispiel in unterschiedlichen Zuständen und
- Figur 13 -: eine weitere Ausführungsform der vorliegenden Erfindung im angelegten Zustand.

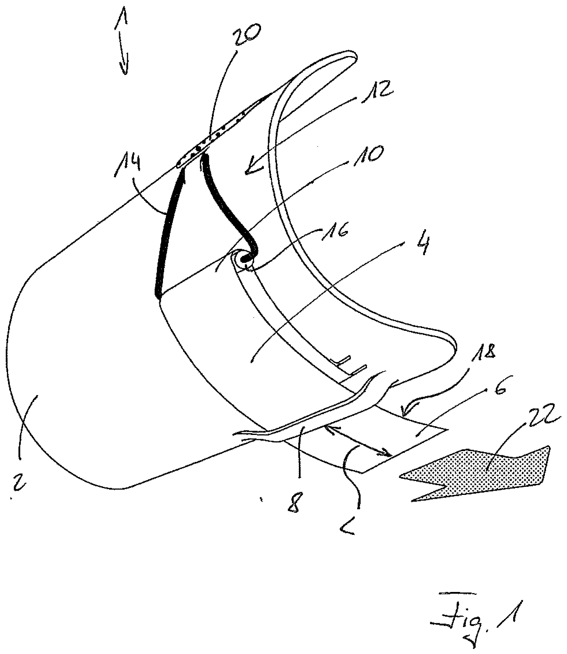

Figur 1 zeigt eine orthopädietechnische Einrichtung 1 gemäß einem ersten Ausführungsbeispiel der vorliegenden Erfindung. Sie verfügt über ein Schalenelement 2, an dem ein Gurt 4 angeordnet ist. Der Gurt verfügt über ein freies Ende 6, das durch ein Führungselement 8 geführt ist. Der Gurt 4 verläuft außen am Schalenelement 2 entlang.

An einem dem freien Ende 6 gegenüberliegenden zweiten Ende 10 befindet sich eine Zugeinrichtung 12, die im gezeigten Ausführungsbeispiel als Gummiband 14 ausgestaltet ist. Selbstverständlich sind auch andere elastische Elemente denkbar. Im gezeigten Ausführungsbeispiel verläuft das Gummiband 14 durch eine Rolle 16, um die das zweite Ende 10 des Gurtes 4 herumgelegt ist. Das zweite Ende 10 entspricht dabei dem Teil des Gurtes 4, der dem freien Ende 6 gegenüberliegt. Dies ist insbesondere der Teil des Gurtes, der vom freien Ende 6 in Längsrichtung des Gurtes 4, also entlang des Gurtes 4, am weitesten entfernt liegt. Das der Gurt 4 an anderer Stelle am Schalenelement 2 befestigt ist, ist für die Definition des zweiten Endes 10 unerheblich.

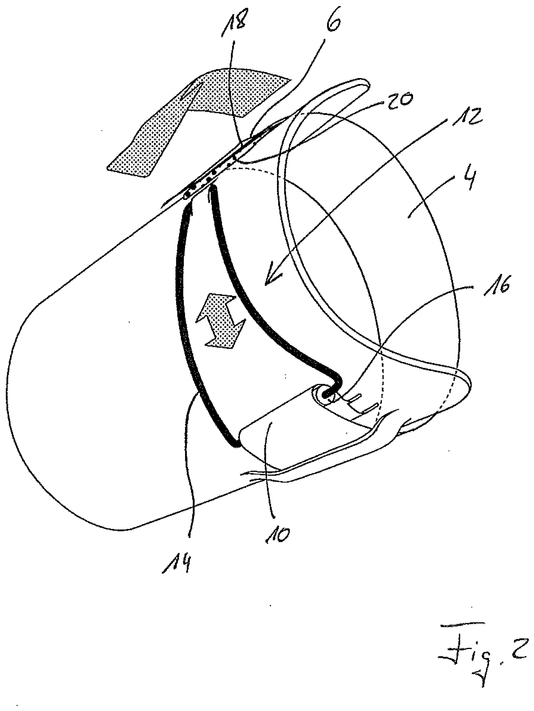

Am freien Ende 6 befindet sich in Figur 1 nicht dargestellt ein Befestigungselement 18, das im gezeigten Ausführungsbeispiel ein Klettverschlusselement ist und an einem Gegenelement 20 angeordnet und befestigt werden kann. Wie jedoch in Figur 1 bereits zu erkennen ist, ist eine freie Länge L, die sich vom freien Ende 6 bis zum Führungselement 8 erstreckt, zu klein, um das Befestigungselement 18 am Gegenelement zu befestigen. Daher muss entlang des Pfeiles 22 eine Zugkraft auf das freie Ende 6 ausgeübt werden, um den Gurt 4 von der in Figur 1 dargestellten zweiten Position in die erste Position zu bringen. Dies ist in Figur 2 dargestellt. Man erkennt, dass der Gurt 4 sich nun in der ersten Position befindet, in der das Gummiband 14 stark gedehnt wurde. Es übt daher eine Zugkraft über die Rolle 16 auf das zweite Ende 10 des Gurtes 4 aus. In der in Figur 2 gezeigten Situation wird jedoch das freie Ende 6 und das darin angeordnete Befestigungselement 18 am Gegenelement 20 angeordnet und befestigt, so dass die von der Zugeinrichtung 12 aufgebrachte Kraft den Gurt 4 nicht aus der in Figur 2 gezeigten ersten Position heraus bewegen kann.

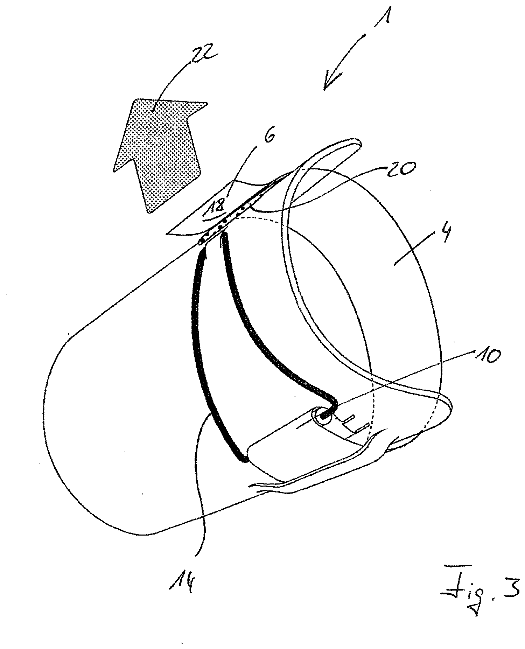

Figur 3 zeigt die Situation zum Ablegen der orthopädietechnischen Einrichtung 1. Das freie Ende 6 des Gurtes 4 wird entlang des Pfeiles 22 bewegt, so dass das Befestigungselement 18 vom Gegenelement 20 gelöst wird. Auch in Figur 3 befindet sich der Gurt noch in der ersten Position, so dass das Gummiband 14 noch immer stark gedehnt ist und eine Kraft auf das zweite Ende 10 des Gurtes 4 ausübt. Die freie Länge ist gegenüber der in Figur 1 gezeigten Situation stark vergrößert. Figur 2 zeigt schematisch das Verschieben des Gurtes 4 aus der ersten Position, die in den Figuren 2 und 3 gezeigt ist, in die in Figur 1 gezeigte zweite Position. Durch die Kombination der beiden Pfeile 24 soll dargestellt werden, dass sich die Zugeinrichtung 12 im vorliegenden Fall also das Gummiband 14 zusammenzieht, so dass das zweite Ende 10 des Gurtes 4 verschoben wird. Dadurch wird auch der Rest des Gurtes durch das Führungselement 8 entlang des Pfeils 22 hindurchgezogen, so dass die freie Länge L wieder reduziert wird. Auf diese Weise ist gewährleistet, dass zum Anlegen der orthopädietechnischen Einrichtung 1 eine ausreichende freie Länge L des Gurtes 4 zur Verfügung steht und gleichzeitig im abgelegten Zustand der Gurt 4 in die zweite Position überführt wird, so dass die freie Länge L stark reduziert ist. Das freie Ende 6 des Gurtes 4 baumelt folglich nicht störend herum und befindet sich immer in einer für den Träger der orthopädietechnischen Einrichtung bekannten und vorteilhafterweise leicht zugänglichen Position.

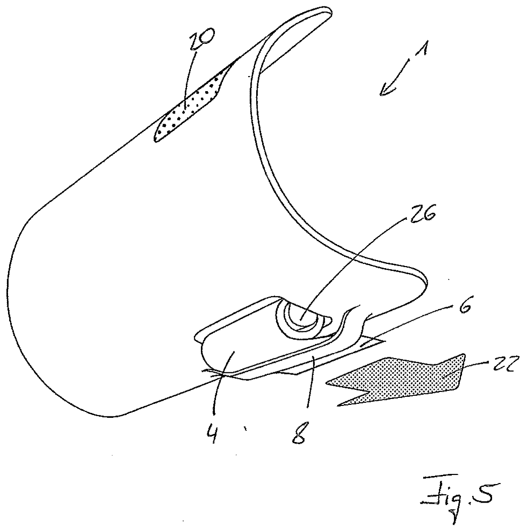

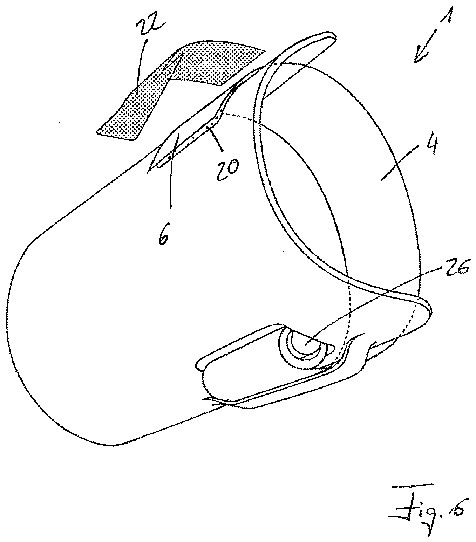

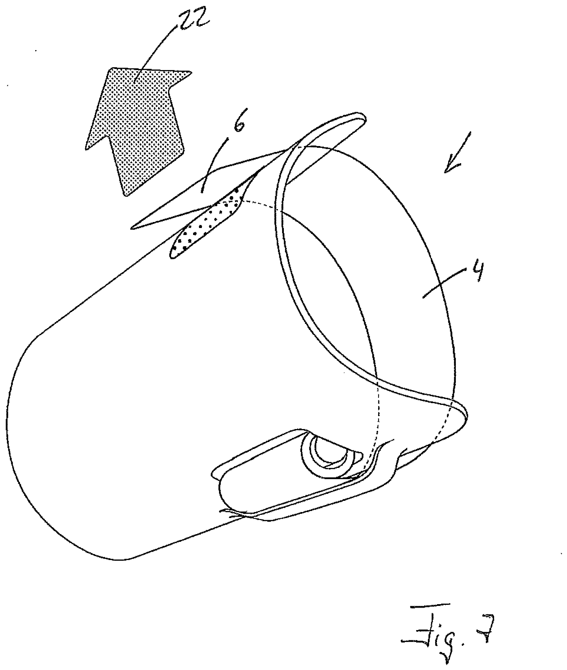

Figur 5 zeigt eine andere Ausgestaltung der orthopädietechnischen Einrichtung 1, bei der der Gurt 4 auf einer Wickeleinrichtung 26 aufgewickelt ist. Figur 5 zeigt die Situation, bei der sich der Gurt in der zweiten Position befindet. Auch hier verfügt der Gurt 4 über das freie Ende 6 durch das Führungselement 8 hindurch geführt. Entlang des Pfeils 22 muss wieder ein Zugkraft auf das freie Ende 6 ausgeübt werden, um den Gurt 4 aus der in Figur 1 gezeigten zweiten Position in die in den Figuren 6 und 7 dargestellte erste Position zu bringen. Erst dann kann das am freien Ende 6 befestigte Befestigungselement 18 am Gegenelement 20 befestigt werden.

Figur 6 zeigt die Situation kurz nach dem Anlegen der orthopädietechnischen Einrichtung 1. Der Gurt 4 erstreckt sich um ein in Figur 6 nicht dargestelltes Körperteil herum und ist entlang des Pfeils 22 bewegt worden. Dabei ist das freie Ende 6 mit dem sich daran befindenden Befestigungselement 18 am Gegenelement 20 befestigt worden. Der Gurt 4 ist von der Wickeleinrichtung 26 abgewickelt worden, so dass sich die freie Länge stark vergrößert hat.

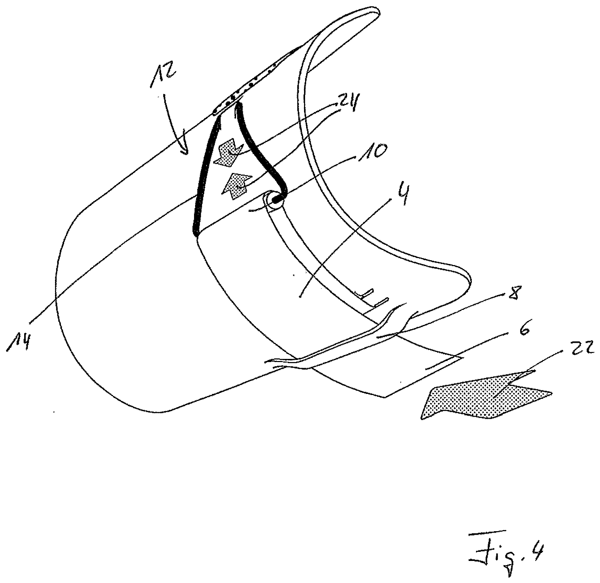

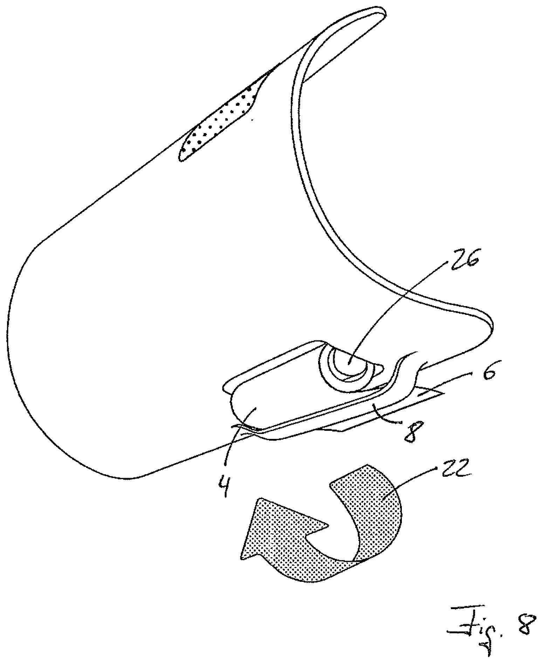

Zum Ablegen oder Lösen der orthopädietechnischen Einrichtung 1 muss, wie in Figur 7 dargestellt, das freie Ende 6 des Gurtes 4 entlang des Pfeiles 22 bewegt werden. Darin unterscheidet sich die Handhabung der in den Figuren 5 bis 8 gezeigten Einrichtung nicht von der Handhabung der in den Figuren 1 bis 4 dargestellten Einrichtung. Durch die von der Wickeleinrichtung 26, die die Zugeinrichtung 12 darstellt, aufgebrachte Zugkraft, wird, wie in Figur 8 dargestellt ist, der Gurt 4 wieder auf die Wickeleinrichtung 26 aufgewickelt, was durch den Pfeil 22 dargestellt werden soll. Das freie Ende 6 befindet sich wieder nah an dem Führungselement 8, so dass die freie Länge L sehr gering ist.

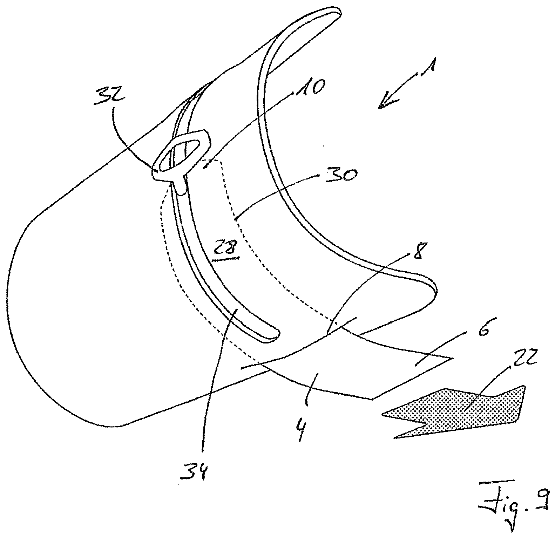

Die Figuren 9 bis 12 zeigen eine weitere Ausgestaltung der orthopädietechnischen Einrichtung 1. Im Gegensatz zu den bisher gezeigten Ausführungsformen verfügt diese Einrichtung jedoch nicht über eine Zugeinrichtung 12, durch die eine Zugkraft auf den Gurt 4 aufgebracht werden könnte. Auch hier ist der Gurt 4 jedoch durch ein Führungselement 8 geführt, das im gezeigten Ausführungsbeispiel ein Schlitz ist. Ein großer Teil des Gurtes 4 befindet sich innerhalb einer Aufnahmeeinrichtung 28 und ist durch die gestrichelte Linie 30 dargestellt. Am zweiten Ende 10 des Gurtes 4 befindet sich ein Griffelement 32, das durch einen Längsschlitz 34 der Aufnahmeeinrichtung 28 hervorsteht.

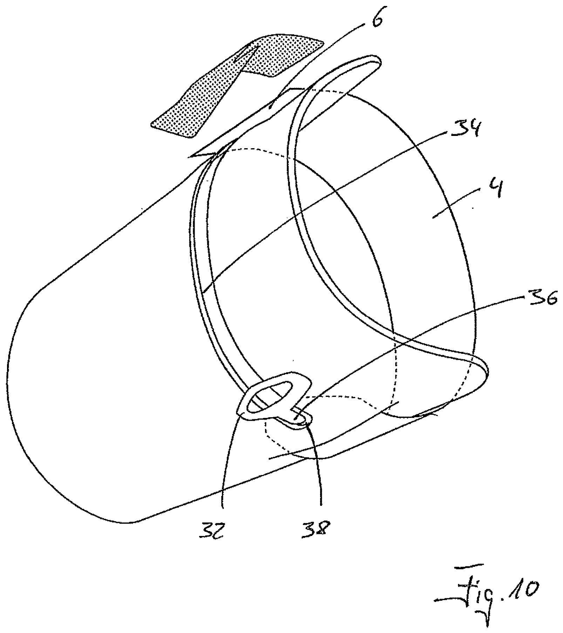

Wird nun entlang des Pfeils 22 eine Zugkraft auf das freie Ende 6 ausgeübt, verschiebt sich der Gurt 4 in die durch den Pfeil 22 gezeigte Richtung und auch das Griffelement 32 gleitet in Längsschlitz 34 in die gezeigte Richtung. Dies geschieht, bis die in Figur 10 gezeigte Situation erreicht ist. Das Griffelement 32 verfügt an seinem unterem Ende über ein Anschlagselement 36, das am Ende des Längsschlitzes 34, der als Anschlag 38 dient, anliegt. Der Gurt 4 hat folglich die erste Position erreicht und das freie Ende 6 kann am Gegenelement 20 angeordnet werden.

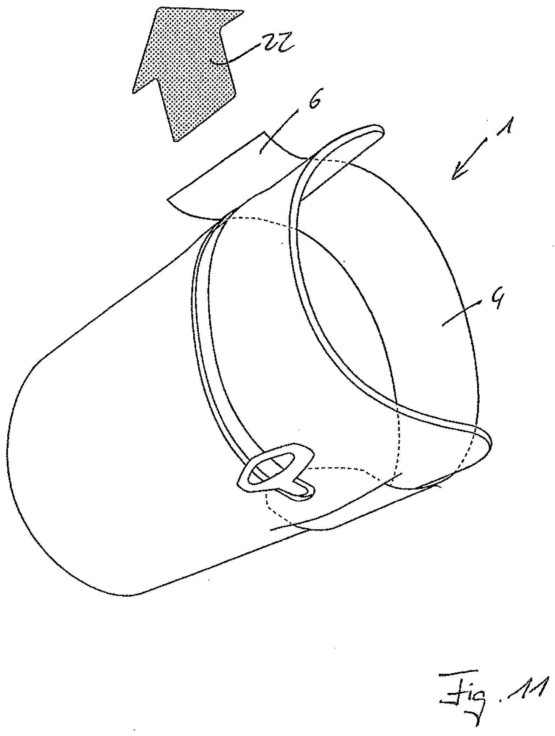

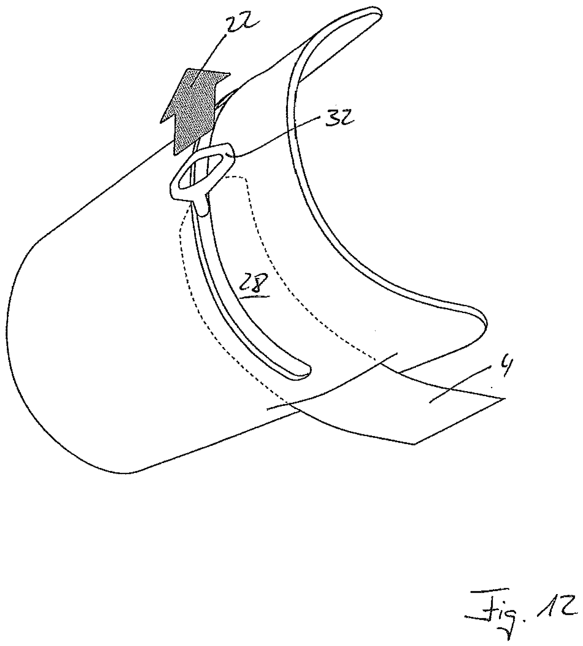

Figur 11 zeigt, was zum Ablegen der orthopädietechnischen Einrichtung 1 vorgenommen werden muss. Das freie Ende 6 des Gurtes 4 muss entlang des Pfeiles 22 wie bei den bereits zuvor gezeigten Ausführungsformen bewegt werden. Da die hier gezeigte Ausführungsform jedoch nicht über eine Zugeinrichtung verfügt, bewegt sich der Gurt 4 zunächst nicht. Figur 12 zeigt, dass entlang des Pfeils 22 eine Zugkraft auf das Griffelement 32 ausgeübt werden muss, so dass der Gurt 4 in die Aufnahmeeinrichtung 28 zurückgezogen wird.

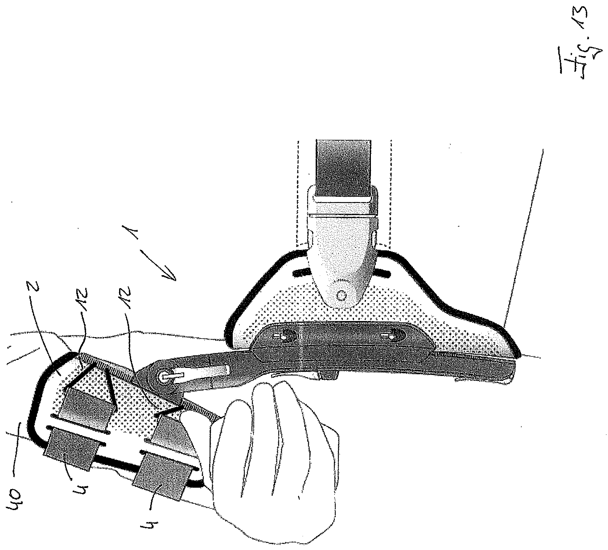

Figur 13 zeigt eine Ausgestaltung einer orthopädietechnischen Einrichtung im angelegten Zustand. Die Einrichtung verfügt über ein Schalenelement 2, das an einem Oberarm 40 des Trägers der Einrichtung 1 angeordnet wird. An dem Schalenelement 2 befinden sich zwei Gurte 4, die sich jeweils in der ersten Position befinden. Beide verfügen über eine Zugeinrichtung 12, die wieder durch ein elastisches Band realisiert wird.

### Bezugszeichenliste

- L: freie Länge
- 1: orthopädietechnische Einrichtung
- 2: Schalenelement
- 4: Gurt
- 6: freies Ende
- 8: Führungselement
- 10: zweites Ende
- 12: Zugeinrichtung
- 14: Gummiband
- 16: Rolle
- 18: Befestigungselement
- 20: Gegenelement
- 22: Pfeil
- 24: Pfeil
- 26: Wickeleinrichtung
- 28: Aufnahmeeinrichtung
- 30: gestrichelte Linie
- 32: Griffelement
- 34: Längsschlitz
- 36: Anschlagselement
- 38: Anschlag
- 40: Oberarm

## Patentansprüche

1. Orthopädietechnische Einrichtung (1) mit
einem Grundkörper, der ein Schalenelement aufweist,
wenigstens einem Gurt (4), der
eingerichtet ist, den Grundkörper an einem Körperteil zu befestigen, durch wenigstens ein Führungselement (8) geführt ist und
ein freies Ende (6) mit einem Befestigungselement (18) zum Befestigen an
einem korrespondierenden Gegenelement (20) aufweist,
wobei sich eine freie Länge (L) des Gurtes (4) von dem freien Ende (6) bis zu dem Führungselement (8) erstreckt,
wobei
der Gurt (4) in eine erste Position und in eine zweite Position bringbar ist, wobei die freie Länge (L) in der zweiten Position nicht ausreicht, um in einem angelegten Zustand der Einrichtung das Befestigungselement (18) an dem Gegenelement (20) zu befestigen, wobei das wenigstens eine Führungselement (8) an dem Grundkörper oder an dem Gurt (4) angeordnet oder darin integriert ist, **dadurch gekennzeichnet, dass** ein Teil des Gurtes (4), der durch das Führungselement (8) bewegt wird, wenn der Gurt (4) in die zweite Position gebracht wird, zumindest nahezu vollständig an dem Schalenelement (2) des Grundkörpers der orthopädietechnischen Einrichtung (1) anliegt und/oder in eine dafür vorgesehene Öffnung oder Hohlraum verschoben und/oder auf eine Wickeleinrichtung (26) aufgewickelt wird.

2. Orthopädietechnische Einrichtung (1) nach Anspruch 1, **dadurch gekennzeichnet, dass** der Grundkörper wenigstens eine Schale oder ein Schienenelement aufweist und das wenigstens eine Führungselement (8) vorzugsweise an der Schale oder dem Schienenelement angeordnet oder darin integriert ist.

3. Orthopädietechnischen Einrichtung (1) nach Anspruch 1 oder 2, **dadurch gekennzeichnet, dass** der Gurt (4) von der ersten Position in die zweite Position bringbar ist, indem eine Zugkraft auf ein zweites Ende (10) des Gurtes (4) aufgebracht wird, das dem freien Ende (6) gegenüberliegt.

4. Orthopädietechnische Einrichtung (1) nach Anspruch 3, **dadurch gekennzeichnet, dass** die Einrichtung (1) eine Zugeinrichtung (12) aufweist, durch die die Zugkraft auf das zweite Ende (10) aufbringbar ist.

5. Orthopädietechnische Einrichtung (1) nach Anspruch 4, **dadurch gekennzeichnet, dass** die Zugeinrichtung (12) wenigstens eine Zugfeder aufweist.

6. Orthopädietechnische Einrichtung (1) nach Anspruch 4 oder 5, **dadurch gekennzeichnet, dass** die Zugeinrichtung (12) eine Wickeleinrichtung (26) aufweist, durch die der Gurt (4) aufwickelbar ist, wobei vorzugsweise das Führungselement (8) nicht Teil der Wickeleinrichtung (26) ist.

7. Orthopädietechnische Einrichtung (1) nach einem der vorstehenden Ansprüche, **dadurch gekennzeichnet, dass** der Gurt (4) von der ersten Position in die zweite Position bringbar ist, indem er zumindest teilweise in eine Aufnahmeeinrichtung (28) verschoben wird.

8. Orthopädietechnische Einrichtung (1) nach Anspruch 7, **dadurch gekennzeichnet, dass** die Aufnahmeeinrichtung (28) mehrere Stege aufweist, die den Gurt (4) quer zu seiner Längsrichtung von dem freien Ende (6) zu dem zweiten Ende (10) überspannen, wenn sich der Gurt (4) in der zweiten Position befindet.

9. Orthopädietechnische Einrichtung (1) nach Anspruch 7 oder 8, **dadurch gekennzeichnet, dass** die Aufnahmeeinrichtung (28) wenigstens einen Längsschlitz (34) entlang der Längsrichtung des Gurtes (4) aufweist, durch den ein Griffelement (32) des Gurtes (4) hindurchragt.

10. Orthopädietechnische Einrichtung (1) nach einem der vorstehenden Ansprüche, **dadurch gekennzeichnet, dass** der Gurt (4) ein Anschlagselement (36) aufweist, das an einem Anschlag (38) der orthopädietechnischen Einrichtung (1) anliegt, wenn sich der Gurt (4) in der ersten Position befindet.

11. Orthopädietechnische Einrichtung (1) nach einem der vorstehenden Ansprüche, **dadurch gekennzeichnet, dass** der Gurt (4) ein Anschlagselement (36) aufweist, das an einem Anschlag (38) der orthopädietechnischen Einrichtung (1) anliegt, wenn sich der Gurt in der zweiten Position befindet.

12. Orthopädietechnische Einrichtung (1) nach Anspruch 10 oder 11, **dadurch gekennzeichnet, dass** eine Position des Anschlags (38) und/oder des Anschlagselementes (36) einstellbar ist.

13. Orthopädietechnische Einrichtung (1) nach einem der vorstehenden Ansprüche, **dadurch gekennzeichnet, dass** sich an dem freien Ende (6) ein Greifelement, beispielsweise eine Schlaufe oder ein Griffstück befindet.

14. Orthopädietechnische Einrichtung (1) nach einem der vorstehenden Ansprüche, **dadurch gekennzeichnet, dass** das wenigstens eine Führungselement (8) lösbar angeordnet und vorzugsweise in unterschiedlichen Positionen, vorteilhafterweise stufenlos an dem Schalen- oder Schienenelement (2) oder dem Gurt (4) befestigbar ist.

## Claims

1. Orthopaedic device (1) with
a main body comprising a shell element,
at least one belt (4) which
is designed to attach the main body to a body part,
is guided through at least one guide element (8) and has a free end (6) with a fastening element (18) for fastening to a corresponding counter-element (20),
wherein a free length (L) of the belt (4) extends from the free end (6) to the guide element (8),
wherein
the belt (4) can be moved into a first position and into a second position, wherein the free length (L) in the second position is insufficient to fasten the fastening element (18) to the counter element (20) in an applied state of the device, wherein the at least one guide element (8) is arranged on the main body or on the belt (4) or integrated therein, **characterised in that** a part of the belt (4) which is moved by the guide element (8) when the belt (4) is moved into the second position, rests at least almost completely against the shell element (2) of the main body of the orthopaedic device (1) and/or is moved into an opening or cavity provided for this purpose and/or is wound onto a winding device (26).

2. Orthopaedic device (1) according to claim 1, **characterised in that** the main body has at least one shell or one rail element and the at least one guide element (8) is preferably arranged on the shell or the rail element or integrated therein.

3. Orthopaedic device (1) according to claim 1 or 2, **characterised in that** the belt (4) can be moved from the first position to the second position by applying a tensile force to a second end (10) of the belt (4) opposite the free end (6).

4. Orthopaedic device (1) according to claim 3, **characterised in that** the device (1) has a tensioning device (12) by means of which the tensile force can be applied to the second end (10).

5. Orthopaedic device (1) according to claim 4, **characterised in that** the tensioning device (12) comprises at least one tension spring.

6. Orthopaedic device (1) according to claim 4 or 5, **characterised in that** the tensioning device (12) has a winding device (26) by means of which the belt (4) can be wound up, preferably with the guide element (8) not being part of the winding device (26).

7. Orthopaedic device (1) according to one of the preceding claims, **characterised in that** the belt (4) can be moved from the first position to the second position by being shifted at least partially into a receiving device (28).

8. Orthopaedic device (1) according to claim 7, **characterised in that** the receiving device (28) has a plurality of webs which span the belt (4) transversely to its longitudinal direction from the free end (6) to the second end (10) when the belt (4) is in the second position.

9. Orthopaedic device (1) according to claim 7 or 8, **characterised in that** the receiving device (28) has at least one longitudinal slot (34) extending in the longitudinal direction of the belt (4), through which a grip element (32) of the belt (4) protrudes.

10. Orthopaedic device (1) according to one of the preceding claims, **characterised in that** the belt (4) has a stop element (36) which rests against a stop (38) of the orthopaedic device (1) when the belt (4) is in the first position.

11. Orthopaedic device (1) according to one of the preceding claims, **characterised in that** the belt (4) has a stop element (36) which rests against a stop (38) of the orthopaedic device (1) when the belt is in the second position.

12. Orthopaedic device (1) according to claim 10 or 11, **characterised in that** a position of the stop (38) and/or the stop element (36) is adjustable.

13. Orthopaedic device (1) according to one of the preceding claims, **characterised in that** a gripping element, for example a loop or a handle, is located at the free end (6).

14. Orthopaedic device (1) according to one of the preceding claims, **characterised in that** the at least one guide element (8) is detachably arranged and preferably can be attached in different positions, preferably steplessly, to the shell or rail element (2) or the belt (4).

## Revendications

1. Dispositif orthopédique (1) comprenant
un corps de base qui comporte un élément formant coque,
au moins une sangle (4) qui
est conçue pour fixer le corps de base à une partie du corps,
est guidée par au moins un élément de guidage (8) et
présente une extrémité libre (6) munie d'un élément de fixation (18) pour la fixation à un élément antagoniste correspondant (20),
dans lequel
une longueur libre (L) de la sangle (4) s'étend depuis l'extrémité libre (6) jusqu'à l'élément de guidage (8),
la sangle (4) peut être amenée dans une première position et dans une deuxième position, la longueur libre (L) dans la deuxième position n'étant pas suffisante pour fixer l'élément de fixation (18) à l'élément antagoniste (20) lorsque le dispositif est en place, ledit au moins un élément de guidage (8) étant disposé sur le corps de base ou sur la sangle (4) ou étant intégré dans celui-ci ou celle-ci,
**caractérisé en ce qu'**une partie de la sangle (4), qui est déplacée par l'élément de guidage (8) lorsque la sangle (4) est amenée dans la deuxième position, s'appuie au moins presque entièrement contre l'élément formant coque (2) du corps de base du dispositif orthopédique (1) et/ou est déplacée jusque dans une ouverture ou cavité prévue à cet effet et/ou est enroulée sur un dispositif d'enroulement (26).

2. Dispositif orthopédique (1) selon la revendication 1,
**caractérisé en ce que** le corps de base comprend au moins une coque ou un élément formant attelle, et, de préférence, ledit au moins un élément de guidage (8) est disposé sur la coque ou sur l'élément formant attelle ou est intégré dans celle-ci ou celui-ci.

3. Dispositif orthopédique (1) selon la revendication 1 ou 2,
**caractérisé en ce que** la sangle (4) peut être amenée de la première position à la deuxième position par application d'une force de traction à une deuxième extrémité (10) de la sangle (4) qui est opposée à l'extrémité libre (6).

4. Dispositif orthopédique (1) selon la revendication 3,
**caractérisé en ce que** le dispositif (1) comprend un dispositif de traction (12) permettant d'appliquer la force de traction à la deuxième extrémité (10).

5. Dispositif orthopédique (1) selon la revendication 4,
**caractérisé en ce que** le dispositif de traction (12) comprend au moins un ressort de traction.

6. Dispositif orthopédique (1) selon la revendication 4 ou 5,
**caractérisé en ce que** le dispositif de traction (12) comprend un dispositif d'enroulement (26) permettant d'enrouler la sangle (4), de préférence, l'élément de guidage (8) ne faisant pas partie du dispositif d'enroulement (26).

7. Dispositif orthopédique (1) selon l'une des revendications précédentes,
**caractérisé en ce que** la sangle (4) peut être amenée de la première position à la deuxième position en étant déplacée au moins partiellement jusque dans un dispositif de réception (28).

8. Dispositif orthopédique (1) selon la revendication 7,
**caractérisé en ce que** le dispositif de réception (28) comprend plusieurs barrettes qui enjambent la sangle (4) transversalement à sa direction longitudinale, de l'extrémité libre (6) à la deuxième extrémité (10), lorsque la sangle (4) se trouve dans la deuxième position.

9. Dispositif orthopédique (1) selon la revendication 7 ou 8,
**caractérisé en ce que** le dispositif de réception (28) présente au moins une fente longitudinale (34) selon la direction longitudinale de la sangle (4), à travers laquelle passe un élément de préhension (32) de la sangle (4).

10. Dispositif orthopédique (1) selon l'une des revendications précédentes,
**caractérisé en ce que** la sangle (4) comprend un élément de butée (36) qui s'appuie contre une butée (38) du dispositif orthopédique (1) lorsque la sangle (4) se trouve dans la première position.

11. Dispositif orthopédique (1) selon l'une des revendications précédentes,
**caractérisé en ce que** la sangle (4) comprend un élément de butée (36) qui s'appuie contre une butée (38) du dispositif orthopédique (1) lorsque la sangle se trouve dans la deuxième position.

12. Dispositif orthopédique (1 ) selon la revendication 10 ou 11,
**caractérisé en ce qu'**une position de la butée (38) et/ou de l'élément de butée (36) est réglable.

13. Dispositif orthopédique (1) selon l'une des revendications précédentes,
**caractérisé en ce qu'**un élément de préhension, par exemple une boucle ou une pièce formant poignée, est situé à l'extrémité libre (6).

14. Dispositif orthopédique (1) selon l'une des revendications précédentes,
**caractérisé en ce que** ledit au moins un élément de guidage (8) est disposé de manière amovible et, de préférence, peut être fixé dans différentes positions, avantageusement de manière continue, à l'élément formant coque ou attelle (2) ou à la sangle (4).
